(19) Europäisches Patentamt
European Patent Office
Office européen des brevets

(11) Veröffentlichungsnummer: **0 303 153 A2**

(12) # EUROPÄISCHE PATENTANMELDUNG

(21) Anmeldenummer: **88112497.8**

(22) Anmeldetag: **01.08.88**

(51) Int. Cl.⁴: **C07D 231/40 , C07D 231/38 , C07C 121/82 , A01N 43/56**

(30) Priorität: **13.08.87 DE 3726919**

(43) Veröffentlichungstag der Anmeldung: **15.02.89 Patentblatt 89/07**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gallenkamp, Bernd, Dr.**
**Paul-Ehrlich-Strasse 13**
**D-5600 Wuppertal 1(DE)**
Erfinder: **Schallner, Otto, Dr.**
**Noldeweg 22**
**D-4019 Monheim(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Unterdorfstrasse 6A**
**D-4000 Düsseldorf 31(DE)**

(54) **1-Arylpyrazole.**

(57) Die Erfindung betrifft neue 1-Arylpyrazole der allgemeinen Formel (I),

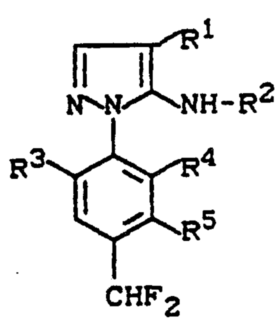

(I)

in welcher

$R^1$ für Wasserstoff oder Nitro steht,

$R^2$ für Wasserstoff oder für einen Rest $-\underset{\underset{O}{\|}}{C}-R^6$ steht,

$R^3$ für Wasserstoff oder Halogen steht,

$R^4$ für Halogen steht und

EP 0 303 153 A2

$R^5$ für Wasserstoff oder Halogen steht,
wobei
$R^6$ für Alkyl, Halogenalkyl, Alkoxyalkyl oder Alkoxyalkoxyalkyl steht, mehrere Verfahren zu ihrer Herstellung sowie ihre Verwendung als Herbizide.

# 1-Arylpyrazole

Die Erfindung betrifft neue 1-Arylpyrazole, mehrere Verfahren zu ihrer Herstellung sowie ihre Verwendung als Herbizide.

Es ist bereits bekannt, daß bestimmte 1-Arylpyrazole, wie beispielsweise das 5-Amino-1-(2,6-dichlor-4-trifluormethylphenyl)-4-nitropyrazol herbizide Eigenschaften besitzen (vgl. z.B. EP 154 115).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue 1-Arylpyrazole der allgemeinen Formel (I),

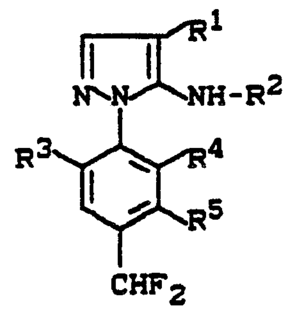

(I)

in welcher
$R^1$ für Wasserstoff oder Nitro steht,
$R^2$ für Wasserstoff oder für einen Rest $-\underset{\underset{O}{\|}}{C}-R^6$ steht,
$R^3$ für Wasserstoff oder Halogen steht,
$R^4$ für Halogen steht und
$R^5$ für Wasserstoff oder Halogen steht,
wobei
$R^6$ für Alkyl, Halogenalkyl, Alkoxyalkyl oder Alkoxyalkoxyalkyl steht,
gefunden.

Weiterhin wurde gefunden, daß man die neuen 1-Arylpyrazole der allgemeinen Formel (I),

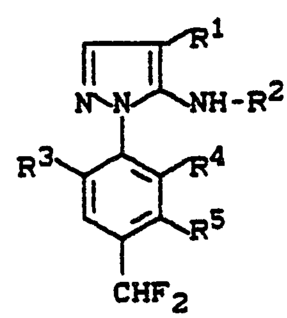

(I)

in welcher
$R^1$ für Wasserstoff oder Nitro steht,
$R^2$ für Wasserstoff oder für einen Rest $-\underset{\underset{O}{\|}}{C}-R^6$ steht,
$R^3$ für Wasserstoff oder Halogen steht,
$R^4$ für Halogen steht und
$R^5$ für Wasserstoff oder Halogen steht,
wobei

$R^6$ für Alkyl, Halogenalkyl, Alkoxyalkyl oder Alkoxyalkoxyalkyl steht, nach einem der im Folgenden beschriebenen Verfahren erhält:

(a) Man erhält 1-Arylpyrazole der Formel (Ia),

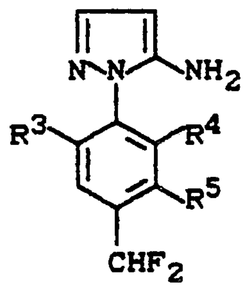

(Ia)

in welcher
$R^3$, $R^4$ und $R^5$ die oben angegebene Bedeutung haben, wenn man N-Aryl-N′-cyanethylhydrazine der Formel (II),

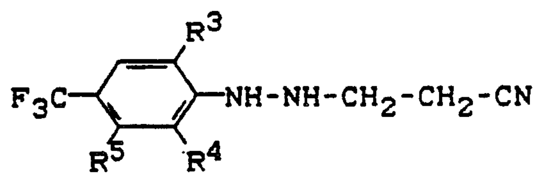

(II)

in welcher
$R^3$, $R^4$ und $R^5$ die oben angegebene Bedeutung haben, mit Natriumhydroxid in Gegenwart eines Verdünnungsmittels cyclisiert;

(b) man erhält 1-Arylpyrazole der Formel (Ib),

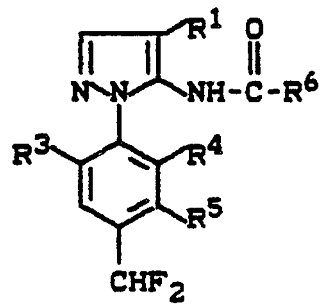

(Ib)

in welcher
$R^1$, $R^3$, $R^4$, $R^5$ und $R^6$ die oben angegebene Bedeutung haben, wenn man die mit Hilfe der erfindungsgemäßen Verfahren (a) oder (c) erhältlichen 1-Arylpyrazole der Formel (Iz),

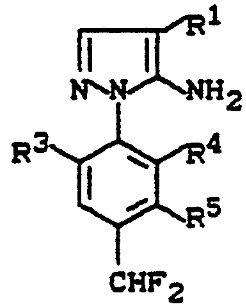

(Iz)

in welcher
$R^1$, $R^3$, $R^4$ und $R^5$ die oben angegebene Bedeutung haben,
mit Acylierungsmitteln der Formel (III),

$$R^6-\underset{\underset{O}{\|}}{C}-A \qquad (III)$$

in welcher
$R^6$ die oben angegebene Bedeutung hat und
A für eine elektronenanziehende Abgangsgruppe steht,
gegebenenfalls in Gegenwart eines Verdünnungsmittels und gegebenenfalls in Gegenwart eines Säurebindemittels umsetzt;

(c) man erhält 1-Arylpyrazole der Formel (Ic),

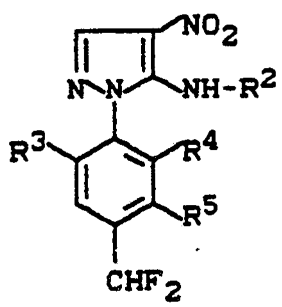

(Ic)

in welcher
$R^2$, $R^3$, $R^4$ und $R^5$ die oben angegebene Bedeutung haben,
wenn man die mit Hilfe der erfindungsgemäßen Verfahren (a) oder (b) erhältlichen 1-Arylpyrazole der Formel (Iy),

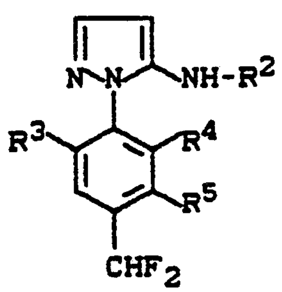

(Iy)

in welcher
$R^2$, $R^3$, $R^4$ und $R^5$ die oben angegebene Bedeutung haben,
mit einem Nitrierungsmittel gegebenenfalls in Gegenwart eines Verdünnungsmittels und gegebenenfalls in Gegenwart eines Reaktionshilfsmittels umsetzt.

Schließlich wurde gefunden, daß die neuen 1-Arylpyrazole der allgemeinen Formel (I) gute herbizide insbesondere auch selektiv herbizide Eigenschaften besitzen.

Überraschenderweise zeigen die erfindungsgemäßen 1-Arylpyrazole bei vergleichbar guter herbizider Wirkung gegenüber Problemunkräutern eine deutlich verbesserte Kulturpflanzenselektivität im Vergleich zu den aus dem Stand der Technik bekannten 1-Aryl-pyrazolen, wie beispielsweise das 5-Amino-4-nitro-1-(2,6-dichlor-4-tri fluormethylphenyl)-pyrazol, welche chemisch und wirkungsmäßig naheliegende Verbindungen sind.

Die erfindungsgemäßen 1-Arylpyrazole sind durch die Formel (I) allgemein definiert. Bevorzugt sind Verbindungen der Formel (I), bei welchen

$R^1$ für Wasserstoff oder Nitro steht,

$R^2$ für Wasserstoff oder für einen Rest $-\underset{\underset{O}{\|}}{C}-R^6$

steht,

$R^3$ für Wasserstoff, Fluor, Chlor oder Brom steht,
$R^4$ für Fluor, Chlor oder Brom steht und
$R^5$ für Wasserstoff, Fluor, Chlor oder Brom steht,

wobei

$R^6$ für jeweils geradkettiges oder verzweigtes Alkyl, Alkoxyalkyl oder Alkoxyalkoxyalkyl mit jeweils 1 bis 6 Kohlenstoffatomen in den einzelnen Alkyl- bzw. Alkoxyteilen oder für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom steht.

Besonders bevorzugt sind Verbindungen der Formel (I), bei welchen
$R^1$ für Wasserstoff oder Nitro steht,
$R^2$ für Wasserstoff oder für einen Rest $-\underset{\underset{O}{\|}}{C}-R^6$

steht,
$R^3$ für Wasserstoff, Fluor oder Chlor steht,
$R^4$ für Fluor oder Chlor steht und
$R^5$ für Wasserstoff, Fluor oder Chlor steht,

wobei

$R^6$ für Methyl, Ethyl, n- oder i-Propyl, n-, i-, s- oder t-Butyl, Methoxymethyl, Ethoxymethyl, n- oder i-Propoxymethyl, Methoxyethyl, Ethoxyethyl, n- oder i-Propoxyethyl, Methoxyethoxyethyl, Ethoxyethoxyethyl, n- oder i-Propoxyethoxy-ethyl, Trifluormethyl, Trichlormethyl, Dichlorfluormethyl, Difluorchlormethyl, Chlormethyl, Iodmethyl, Brommethyl, Dichlormethyl, 1-Chlorethyl, 2-Chlorethyl, 2-Bromethyl, 3-Chlorpropyl, Pentafluorethyl oder Heptafluorpropyl steht.

Ganz besonders bevorzugt sind Verbindungen oder Formel (I), bei welchen
$R^1$ für Nitro steht,
$R^2$ für Wasserstoff oder für einen Rest $-\underset{\underset{O}{\|}}{C}-R^6$

steht,
$R^3$ für Wasserstoff, Fluor oder Chlor steht,
$R^4$ für Fluor oder Chlor steht und
$R^5$ für Wasserstoff, Fluor oder Chlor steht,

wobei

$R^6$ für Methyl, Ethyl, n- oder i-Propyl, Methoxymethyl, Ethoxymethyl, Methoxyethyl, Ethoxyethyl, Methoxyethoxyethyl, Ethoxyethoxyethyl, Trifluormethyl, Chlormethyl, Dichlormethyl, 1-Chlorethyl, 2-Chlorethyl, Pentafluorethyl oder Heptafluorpropyl steht.

Verwendet man beispielsweise N-(2,6-Dichlor-4-difluormethylphenyl)-N′-(2-cyanethyl)-hydrazin als Ausgangsverbindung, so läßt sich der Reaktionsablauf des erfindungsgemäßen Verfahrens (a) durch das folgende Formelschema darstellen:

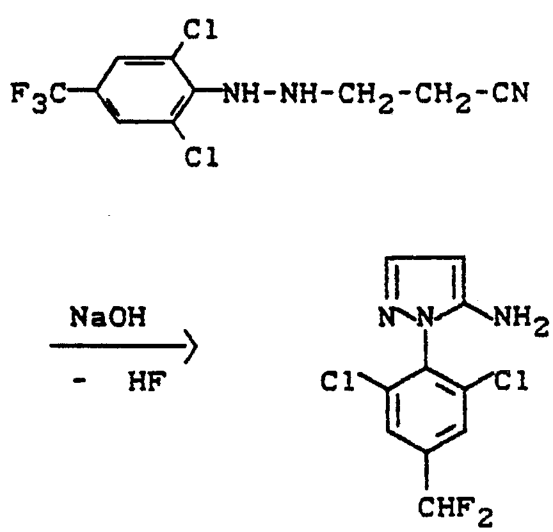


Verwendet man beispielsweise 5-Amino-1-(2,3,6-trichlor-4-difluormethylphenyl)-pyrazol und Acetanhydrid als Ausgangsstoffe, so läßt sich der Reaktionsablauf des erfindungsgemäßen Verfahrens (b) durch das folgende Formelschema darstellen:

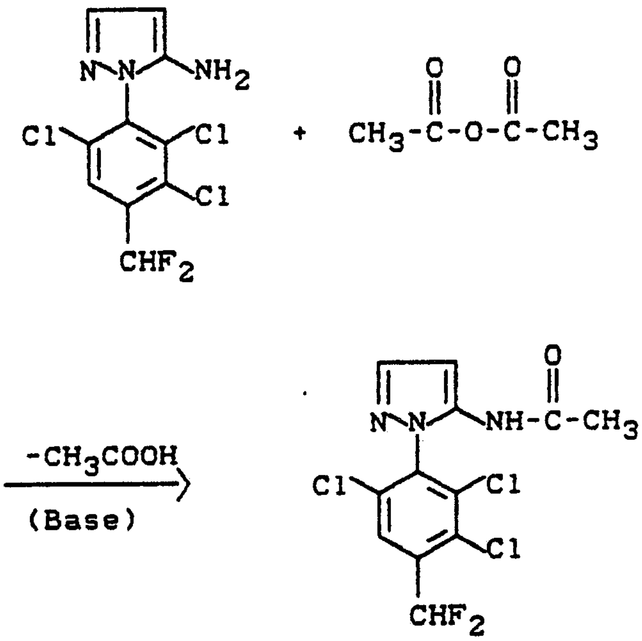


Verwendet man beispielsweise 5-Acetamido-1-(2,6-dichlor-4-difluormethylphenyl)-pyrazol als Ausgangsverbindung, so läßt sich der Reaktionsablauf des erfindungsgemäßen Verfahrens (c) durch das folgende Formelschema darstellen:

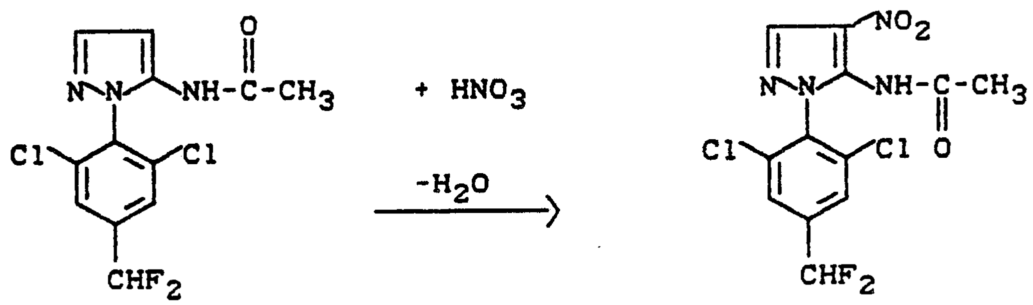


Die zur Durchführung des erfindungsgemäßen Verfahrens (a) als Ausgangsstoffe benötigten N-Aryl-N′-cyanethylhydrazine sind durch die Formel (II) allgemein definiert. In dieser Formel (II) stehen $R^3$, $R^4$ und $R^5$ vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die N-Aryl-N′-cyanethylhydrazine der Formel (II) sind noch nicht bekannt.

Man erhält sie, wenn man 1-Arylhydrazine der Formel (IV),

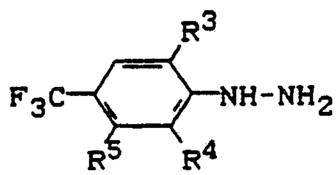 (IV)

in welcher

$R^3$, $R^4$ und $R^5$ die oben angegebene Bedeutung haben,

mit Acrylnitril der Formel (V),

$CH_2=CH\text{-}CN$ (V)

gegebenenfalls in Gegenwart eines Verdünnungsmittels wie beispielsweise Ethanol bei Temperaturen zwischen 50 °C und 150 °C umsetzt.

Die Arylhydrazine der Formel (IV) sind bekannt oder erhältlich in Analogie zu bekannten Verfahren (vgl. z.B. DE-OS 3 226 513, DE-OS 3 226 496 oder EP 224 831).

Das Acrylnitril der Formel (V) ist eine allgemein bekannte Verbindung der organischen Chemie.

Die zur Durchführung des erfindungsgemäßen Verfahrens (b) als Ausgangsstoffe benötigten 1-Arylpyrazole sind durch die Formel (Iz) allgemein definiert. In dieser Formel (Iz) stehen $R^1$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Arylpyrazole der Formel (Iz) sind erfindungsgemäße Verbindungen und erhältlich mit Hilfe der erfindungsgemäßen Verfahren (a) oder (c).

Die zur Durchführung des erfindungsgemäßen Verfahrens (b) weiterhin als Ausgangsstoffe benötigten Acylierungsmittel sind durch die Formel (III) allgemein definiert. In dieser Formel (III) steht $R^6$ vorzugsweise für diejenigen Reste, die bereits im Zusammenhang mit der Beschreibung der erfindungsgemäßen Stoffe der Formel (I) als bevorzugt für diesen Substituenten genannt wurden.

A steht vorzugsweise für Halogen, insbesondere für Chlor oder Brom, oder für einen Rest $R^6\text{-}\underset{\underset{O}{\|}}{C}\text{-O-}$,

wobei

$R^6$ die oben angegebene Bedeutung hat.

Die Acylierungsmittel der Formel (III) sind allgemein bekannte Verbindungen der organischen Chemie.

Die zur Durchführung des erfindungsgemäßen Verfahrens (c) als Ausgangsstoffe benötigten 1-Arylpyrazole sind durch die Formel (Iy) allgemein definiert. In dieser Formel (Iy) stehen $R^2$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Arylpyrazole der Formel (Iy) sind erfindungsgemäße Verbindungen und erhältlich mit Hilfe der erfindungsgemäßen Verfahren (a) oder (b).

Als Verdünnungsmittel zur Durchführung des erfindungsgemäßen Verfahrens (a) kommen übliche organische Lösungsmittel infrage. Vorzugsweise verwendet man Alkohole, wie beispielsweise Methanol, Ethanol oder Propanol oder deren Gemische mit Wasser.

Die Reaktionstemperaturen können bei der Durchführung des erfindungsgemäßen Verfahrens (a) in einem größeren Bereich variiert werden. Im allgemeinen arbeitet man bei Temperaturen zwischen 30 °C und 150 °C, vorzugsweise bei Temperaturen zwischen 50 °C und 120 °C.

Zur Durchführung des erfindungsgemäßen Verfahrens (a) setzt man pro Mol an N-Aryl-N′-cyanethylhydrazin der Formel (II) im allgemeinen 1.0 bis 5.0 Mol, vorzugsweise 1.5 bis 3.0 Mol an Natriumhydroxid ein.

Man erhitzt die Reaktionskomponenten in einem geeigneten Verdünnungsmittel für mehrere Stunden auf die erforderliche Reaktionstemperatur und kontrolliert dabei die Umsetzung dünnschichtchromatographisch. Die Aufarbeitung und Isolierung der Reaktionsprodukte der Formel (Ia) erfolgt nach allgemein üblichen Methoden.

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetra chlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) wird gegebenenfalls in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in

einem größeren Bereich variiert werden. Im allgemeinen arbeitet man bei Temperaturen zwischen - 20 °C und 120 °C, vorzugsweise bei Temperaturen zwischen 0 °C und 80 °C.

Zur Durchführung des erfindungsgemäßen Verfahrens (b) setzt man pro Mol an 1-Arylpyrazol der Formel (Iz) im allgemeinen 1.0 bis 3.0 Mol, vorzugsweise 1.0 bis 1.5 Mol an Acylierungsmittel der Formel (III) und gegebenenfalls 1.0 bis 3.0 Mol, vorzugsweise 1.0 bis 1.5 Mol an Säurebindemittel ein.
Die Reaktionsdurchführung, Aufarbeitung und Isolierung der Reaktionsprodukte erfolgt nach allgemein üblichen Methoden.

Als Nitrierungsmittel zur Durchführung des erfindungsgemäßen Verfahrens (c) kommen alle üblichen Nitrierungsmittel infrage. Vorzugsweise verwendet man konzentrierte Salpetersäure oder Nitriersäure.

Als Verdünnungmittel zur Durchführung des erfindungsgemäßen Verfahrens (c) kommen alle üblicherweise für derartige elektrophile Substitutionen verwendbaren Lösungsmittel infrage. Vorzugsweise verwendet man die als Reagenzien infrage kommenden Säuren oder Gemische, wie beispielsweise Salpetersäure oder Nitriersäure gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungmittel infrage.

Als Katalysatoren oder Reaktionshilfsmittel zur Durchführung des erfindungsgemäßen Verfahrens (c) kommen ebenfalls die für derartige Reaktionen üblichen Katalysatoren infrage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des erfindungsgemäßen Verfahrens (c) in einem größeren Bereich variiert werden. Im allgemeinen arbeitet man zwischen - 50 °C und + 200 °C, vorzugsweise zwischen - 20 °C und + 150 °C.

Zur Durchführung des erfindungsgemäßen Verfahrens (c) setzt man pro Mol an 1-Aryl-pyrazol der Formel (Iy) im allgemeinen 1,0 bis 10,0 Mol, vorzugsweise 1,0 bis 5,0 Mol an Nitrierungsmittel und gegebenenfalls 0,1 bis 10 Mol an Katalysator oder Reaktionshilfsmittel ein. Die Reaktionsführung, Aufarbeitung und Isolierung der Reaktionsprodukte der Formel (Ic) erfolgt in allgemein üblicher Art und Weise. Je nach Säurekonzentration, Art des gewählten Verdünnungsmittels, Reaktionstemperatur und Reaktionsdauer ist es dabei möglich, daß eventuell vorhandene Acylgruppen an dem Aminosubstituenten in der 5-Position des Pyrazolringes entweder erhalten bleiben oder im Verlaufe der Nitrierungsreaktion gleichzeitig abgespalten werden (vgl. hierzu auch die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.
<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von dikotylen Unkräutern in monokotylen und dikotylen Kulturen, wie beispielsweise Weizen, Mais, Reis oder Soja einsetzen.

In entsprechenden Aufwandmengen besitzen die erfindungsgemäßen Wirkstoffe darüberhinaus auch

wachstumsregulatorische Wirkung und lassen sich beispielsweise als Defoliantien einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, infrage. Auch Mischungen mit 2,4-Dichlorphenoxyessigsäure;
2,4-Dichlorphenoxypropionsäure;
4-(2,4-Dichlorphenoxy)-buttersäure;
(2-Methyl-4-chlorphenoxy)-essigsäure;
(4-Chlor-2-methylphenoxy)-propionsäure;
3,5,6-Trichlor-2-pyridyloxyessigsäure;
2-[4-(2,4-Dichlorphenoxy)-phenoxy]-propionsäure, deren Methyl- oder deren Ethylester;
2-{4-[(6-Chlor-2-benzoxazolyl)-oxy]-phenoxy}-propansäure, deren Methyl- oder deren Ethylester;
Butyl-2-[4-(5-trifluormethyl-2-pyridinyloxy)-phenoxy]-propanoat;
2-[4-(3,5-Dichlorpyrid-2-yloxy)-phenoxy]-propionsäure-(trimethylsilylmethylester);
2-{-4-[(3-Chlor-5-(trifluormethyl)-2-pyridinyl)-oxy]-phenoxy}-propansäure bzw. -propansäureethylester;
3,6-Dichlor-2-pyridincarbonsäure;
Chloressigsäure-N-(methoxymethyl)-2,6-diethylanilid;
N-(Butoxymethyl)-2-chlor-N-(2,6-diethylphenyl)-acetamid;
2-Chlor-N-(2,6-dimethylphenyl)-N-[(1H)-pyrazol-1-yl-methyl]-acetamid;
2-Ethyl-6-methyl-N-(1-methyl-2-methoxyethyl)-chloracetanilid;

α-Chlor-2′,6′-diethyl-N-(2-propoxyethyl)-acetanilid;
2-Chlor-N-isopropylacetanilid;
4-(Di-n-propylamino)-3,5-dinitrobenzolsulfonamid;
N-(1-Ethylpropyl)-3,4-dimethyl-2,6-dinitroanilin;
2,6-Dinitro-4-trifluormethyl-N,N-dipropylanilin;
5-(2-Chlor-4-trifluormethyl-phenoxy)-2-nitro-benzoesäure;
Methyl-5-(2,4-dichlorphenoxy)-2-nitrobenzoat;
5-(2-Chlor-4-trifluormethyl-phenoxy)-N-methylsulfonyl-2-nitrobenzamid;
(2-Ethoxy-1-methyl-2-oxo-ethyl)-5-[2-chlor-4-(trifluormethyl)-phenoxy]-2-nitrobenzoat;
N,N-Dimethyl-N′-(3-chlor-4-methylphenyl)-harnstoff;
N,N-Dimethyl-N′-(4-isopropylphenyl)-harnstoff;
2-[1-(Ethoximino)-butyl]-3-hydroxy-5-[tetrahydro-(2H)-thiopyran-3-yl]-2-cyclohexen-1-on;
2-[1-(Ethoxamino)-butyliden]-5-(2-ethylthiopropyl)-1,3-cyclohexadion;
2-[4,5-Dihydro-4-methyl-4-isopropyl-5-oxo-(1H)-imidazol-2-yl]-5-ethyl-pyridin-3-carbonsäure;
Methyl-2-[4,5-dihydro-4-methyl-4-(1-methylethyl)-5-oxo-1H-imidazol-2-yl]-4(5)-methylbenzoat;
2-[5-Methyl-5-(1-methylethyl)-4-oxo-2-imidazolin-2-yl]-3-chinolincarbonsäure;
3,5-Dibrom-4-hydroxy-benzonitril;
3,5-Diiod-4-hydroxybenzonitril;
N-Methyl-2-(1,3-benzthiazol-2-yloxy)-acetanilid;
2-[[[[(4,6-Dimethoxypyrimidin-2-yl)-amino]-carbonyl]-amino]-sulfonyl]-methyl]-benzoesäuremethylester;
Ethyl-2-{[(4-chlor-6-methoxy-2-pyrimidinyl)-aminocarbonyl]-aminosulfonyl}-benzoat;
2-Chlor-N-{[(4-methoxy-6-methyl-1,3,5-triazin-2-yl)-amino]-carbonyl}-benzolsulfonamid;
2-{[[(4-Methoxy-6-methyl-1,3,5-triazin-2-yl)-amino)-carbonyl]-amino]-sulfonyl}-benzoesäure oder deren Methylester;
Methyl-2-{[(4,6-dimethyl-2-pyrimidinyl)-aminocarbonyl]-aminosulfonyl}-benzoat;
3-[[[[(4-Methoxy-6-methyl-1,3,5-triazin-2-yl)-amino]-carbonyl]-amino]-sulfonyl]-thiophen-2-carbonsäuremethylester;
N,N-Di-n-propyl-thiocarbamidsäure-S-ethylester;
S-Ethyl-N,N-hexamethylen-thiolcarbamat;
S-[(4-Chlorphenyl)-methyl]-N,N-diethyl-thiocarbamat;
N,N-Diisopropyl-S-(2,3,3-trichlorallyl)-thiolcarbamat;
2-Chlor-4-ethylamino-6-isopropylamino-1,3,5-triazin;
2-Chlor-4-ethylamino-6-(3-cyanopropylamino)-1,3,5-triazin;
2,4-Bis-[N-ethylamino]-6-methylthio-1,3,5-triazin;
4-Ethylamino-2-t-butylamino-6-methylthio-s-triazin;
4-Amino-6-t-butyl-3-ethylthio-1,2,4-triazin-5(4H)-on;
3-Isopropyl-2,1,3-benzothiadiazin-4-on-2,2-dioxid;
exo-1-Methyl-4-(1-methylethyl)-2-(2-methylphenyl-methoxy)-7-oxabicyclo-(2,2,1)-heptan und 0-(6-Chlor-3-phenylpyridazin-4-yl)-S-octyl-thiocarbonat sind gegebenenfalls von Vorteil.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdunnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

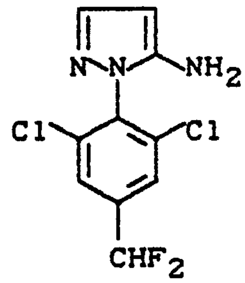

(Verfahren a)

29,8 g (0,1 Mol) N-(2,6-Dichlor-4-trifluormethylphenyl)-N′-(2-cyanethyl)-hydrazin in 200 ml Ethanol werden mit 18 g (0.2 Mol) 45 %iger technischer Natronlauge 2 Stunden auf Rückflußtemperatur erhitzt. Nach beendeter Reaktion (dünnschichtchromatographische Kontrolle; Dichlormethan/Methanol 19 : 1) engt man im Vakuum ein, nimmt den Rückstand in Dichlormethan/Wasser auf, trennt die organische Phase ab, wäscht mit Wasser, trocknet über Natriumsulfat und entfernt das Lösungsmittel im Vakuum. Nach Umkristallisation aus 500 ml Toluol erhält man 16 g (58 % der Theorie) an 5-Amino-1-(2,6-dichlor-4-difluormethylphenyl)-pyrazol vom Schmelzpunkt 110 °C -111 °C.
$^{19}F$-NMR ($CDCl_3$) : $\delta$ = 34.3 (d, 2F) ppm.

Beispiel 2

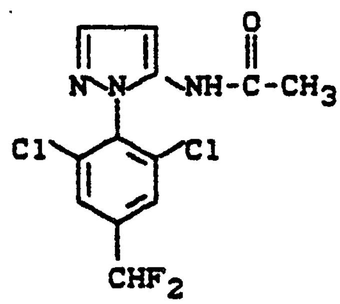

(Verfahren b)

Zu 5,56 g (0,02 Mol) 5-Amino-1-(2,6-dichlor-4-difluormethylphenyl)-pyrazol in 40 ml Toluol tropft man bei 20 °C unter Rühren 2 ml (0,021 Mol) Acetanhydrid, rührt nach beendeter Zugabe weitere 16 Stunden bei Raumtemperatur, saugt den ausgefallenen Niederschlag ab und trocknet den Rückstand.

Man erhält 5,4 g (84 % der Theorie) an 5-Acetamido-1-(2,6-dichlor-4-difluormethylphenyl)-pyrazol von Schmelzpunkt 209 °C -211 °C.

Beispiel 3

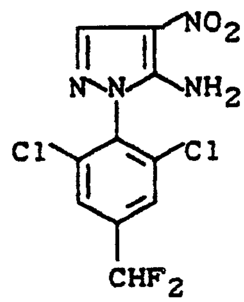

(Verfahren c)

Zu 4,8 g (0,015 Mol) 5-Acetamido-1-(2,6-dichlor-4-difluormethylphenyl)-pyrazol in 40 ml konzentrierter Schwefelsäure gibt man bei - 20 °C tropfenweise unter Rühren 1.07 ml 98 %ige Salpetersäure, rührt nach beendeter Zugabe 4 Stunden bei 20 °C, gießt die Reaktionsmischung auf Eis, saugt den ausgefallenen Feststoff ab, löst ihn in Dichlormethan, wäscht je einmal mit Wasser und wässriger Natriumhydrogencarbonatlösung, trocknet über Natriumsulfat und entfernt das Lösungsmittel im Vakuum.

Man erhält 4,0 g (83 % der Theorie) an 5-Amino-1-(2,6-dichlor-4-difluormethylphenyl)-4-nitro-pyrazol vom Schmelpunkt 165 °C - 168 °C.

In entsprechender Weise und gemäß den allgemeinen Angaben zur Herstellung erhält man die folgenden 1-Aryl-pyrazole der allgemeinen Formel (I):

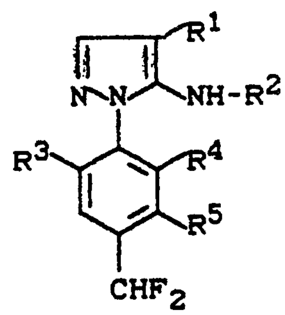

(I)

T a b e l l e 1

| Bsp. Nr. | $R^1$ | $R^2$ | $R^3$ | $R^4$ | $R^5$ | Schmelzpunkt/°C |
|---|---|---|---|---|---|---|
| 4 | H | H | Cl | Cl | Cl | $^1$H-NMR*): 5,2; 5,4; 7,25; 7,35; 7,9 |
| 5 | H | $-\overset{O}{\overset{\|}{C}}-CHCl_2$ | Cl | Cl | H | 91 - 92 |
| 6 | $NO_2$ | $-\overset{O}{\overset{\|}{C}}-CHCl_2$ | Cl | Cl | H | 104 - 106 |
| 7 | $NO_2$ | $-CO-\underset{Cl}{\underset{\vert}{CH}}-CH_3$ | Cl | Cl | H | 107 - 113 |
| 8 | $NO_2$ | $-CO-CH_3$ | Cl | Cl | H | $^1$H-NMR*): 8,35 |
| 9 | $NO_2$ | $-CO-\underset{Cl}{\underset{\vert}{CH}}-C_2H_5$ | Cl | Cl | H | 79 - 87 |

*) Das $^1$H-NMR-Spektrum wurde in Hexadeutero-Dimethylsulfoxid ($DMSO-d_6$) mit Tetramethylsilan (TMS) als innerem Standard aufgenommen. Angegeben ist die chemische Verschiebung als δ-Wert in ppm.

Herstellung der Ausgangsverbindungen

Beispiel II-1

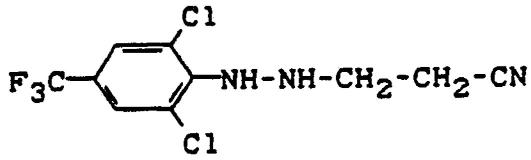

24,5 g (0,1 Mol) 2,6-Dichlor-4-trifluormethyl-phenylhydrazin und 6 g (0,11 Mol) Acrylnitril werden in 100 ml Ethanol 4 Tage auf Rückflußtemperatur erhitzt. Nach Entfernen der flüchtigen Bestandteile im Vakuum erhält man 30 g (99 % der Theorie) an N-(2,6-Dichlor-4-trifluormethylphenyl)-N-(2-cyanethyl)-hydrazin als Öl.

$^1$H-NMR ($CDCl_3$/TMS): $\delta$ = 2,55; 3,1; 4,55; 6,0; 7,55 ppm.

In entsprechender Weise erhält man

Bsp. II-2:

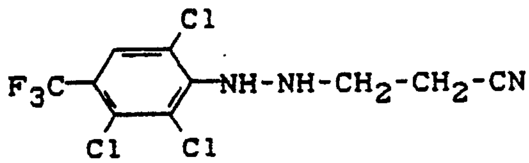

Fp. 47 °C - 49 °C

Anwendungsbeispiele

In den folgenden Anwendungsbeispielen wurde die nachstehend aufgeführte Verbindung als Vergleichsubstanz eingesetzt:

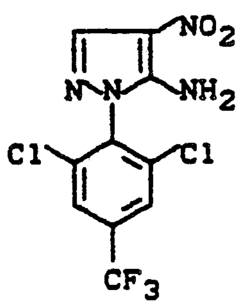

(A)

5-Amino-1-(2,6-dichlor-4-trifluormethylphenyl)-4-nitro-pyrazol bekannt aus EP 154 115/Beispiel Nr. 50).

Beispiel A

Pre-emergence-Test

Lösungsmittel: 5-Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Nutzpflanzenselektivität bei vergleichbarer herbizider Wirksamkeit gegenüber der Vergleichssubstanz (A) zeigen in diesem Test z.B. die Verbindungen gemäß folgender Herstellungsbeispiele: 3, 6 und 7.

Beispiel B

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der Nutzpflanzenselektivität bei Vergleichbarer herbizider Wirksamkeit gegenüber der Vergleichssubstanz (A) zeigen in diesem Test z.B. die Verbindungen gemäß folgender Herstellungsbeispiele: 3 und 6.

**Ansprüche**

1. 1-Arylpyrazole der allgemeinen Formel (I)

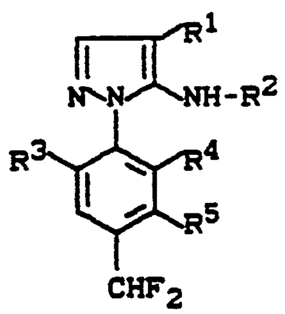

(I)

in welcher

$R^1$ für Wasserstoff oder Nitro steht,

$R^2$ für Wasserstoff oder für einen Rest $-\underset{\underset{O}{\|}}{C}-R^6$ steht,

$R^3$ für Wasserstoff oder Halogen steht,

$R^4$ für Halogen steht und
$R^5$ für Wasserstoff oder Halogen steht,
wobei
$R^6$ für Alkyl, Halogenalkyl, Alkoxyalkyl oder Alkoxyalkoxyalkyl steht.

2. 1-Arylpyrazole der Formel (I) gemäß Anspruch 1, in welcher
$R^1$ für Wasserstoff oder Nitro steht,
$R^2$ für Wasserstoff oder für einen Rest $-\underset{\underset{O}{\|}}{C}-R^6$
steht,
$R^3$ für Wasserstoff, Fluor, Chlor oder Brom steht,
$R^4$ für Fluor, Chlor oder Brom steht und
$R^5$ für Wasserstoff, Fluor, Chlor oder Brom steht,
wobei
$R^6$ für jeweils geradkettiges oder verzweigtes Alkyl, Alkoxyalkyl oder Alkoxyalkoxyalkyl mit jeweils 1 bis 6 Kohlenstoffatomen in den einzelnen Alkyl-bzw. Alkoxyteilen oder für geradkettiges oder verzweigtes Halogenalkyl mit 1 bis 6 Kohlenstoffatomen und 1 bis 9 gleichen oder verschiedenen Halogenatomen steht.

3. 1-Arylpyrazole der Formel (I) gemäß Anspruch 1, in welcher
$R^1$ für Wasserstoff oder Nitro steht,
$R^2$ für Wasserstoff oder für einen Rest $-\underset{\underset{O}{\|}}{C}-R^6$
steht,
$R^3$ für Wasserstoff, Fluor oder Chlor steht,
$R^4$ für Fluor oder Chlor steht und
$R^5$ für Wasserstoff, Fluor oder Chlor steht,
wobei
$R^6$ für Methyl, Ethyl, n- oder i-Propyl, n-, i-, s- oder t-Butyl, Methoxymethyl, Ethoxymethyl, n- oder i-Propoxymethyl, Methoxyethyl, Ethoxyethyl, n- oder i-Propoxyethyl, Methoxyethoxyethyl, Ethoxyethoxyethyl, n- oder i-Propoxyethoxy-ethyl, Trifluormethyl, Trichlormethyl, Dichlorfluormethyl, Difluorchlormethyl, Chlormethyl, Iodmethyl, Brommethyl, Dichlormethyl, 1-Chlorethyl, 2-Chlorethyl, 2-Bromethyl, 3-Chlorpropyl, Pentafluorethyl oder Heptafluorpropyl steht.

4. Verfahren zur Herstellung von 1-Arylpyrazolen der allgemeinen Formel (I),

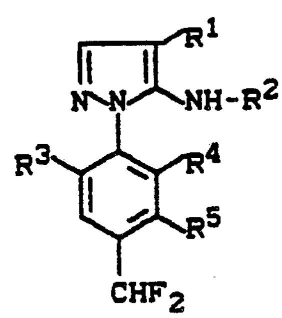

(I)

in welcher
$R^1$ für Wasserstoff oder Nitro steht,
$R^2$ für Wasserstoff oder für einen Rest $-\underset{\underset{O}{\|}}{C}-R^6$
steht,
$R^3$ für Wasserstoff oder Halogen steht,
$R^4$ für Halogen steht und
$R^5$ für Wasserstoff oder Halogen steht,
wobei
$R^6$ für Alkyl, Halogenalkyl, Alkoxyalkyl oder Alkoxyalkoxyalkyl steht.
dadurch gekennzeichnet, daß man
(a) 1-Arylpyrazole der Formel (Ia),

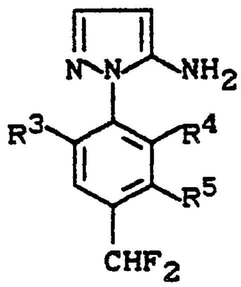

(Ia)

in welcher
$R^3$, $R^4$ und $R^5$ die oben angegebene Bedeutung haben,
erhält, wenn man N-Aryl-N′-cyanethylhydrazine der Formel (II),

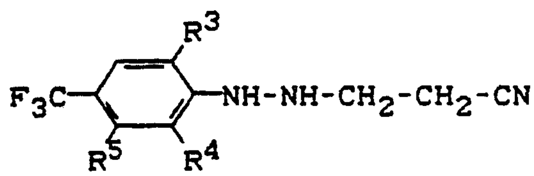

(II)

in welcher
$R^3$, $R^4$ und $R^5$ die oben angegebene Bedeutung haben,
mit Natriumhydroxid in Gegenwart eines Verdünnungsmittels cyclisiert;
oder daß man
(b) 1-Arylpyrazole der Formel (Ib),

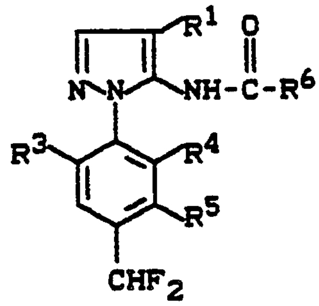

(Ib)

in welcher
$R^1$, $R^3$, $R^4$, $R^5$ und $R^6$ die oben angegebene Bedeutung haben,
erhält, wenn man die mit Hilfe der erfindungsgemäßen Verfahren (a) oder (c) erhältlichen 1-Arylpyrazole der Formel (Iz),

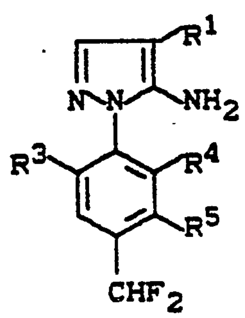

(Iz)

in welcher
$R^1$, $R^3$, $R^4$ und $R^5$ die oben angegebene Bedeutung haben,

mit Acylierungsmitteln der Formel (III),

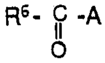

(III)

in welcher
$R^6$ die oben angegebenen Bedeutung hat und
A für eine elektronenanziehende Abgangsgruppe steht,
gegebenenfalls in Gegenwart eines Verdünnungsmittels und gegebenenfalls in Gegenwart eines Säurebindemittels umsetzt; oder daß man
(c) 1-Arylpyrazole der Formel (Ic),

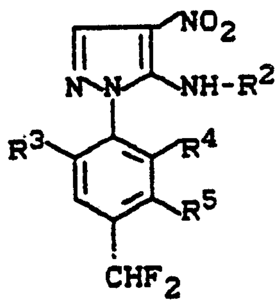

(Ic)

in welcher
$R^2$, $R^3$, $R^4$ und $R^5$ die oben angegebene Bedeutung haben,
erhält, wenn man die mit Hilfe der erfindungsgemäßen Verfahren (a) oder (b) erhältlichen 1-Arylpyrazole der Formel (Iy),

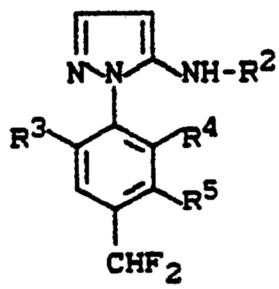

(Iy)

in welcher
$R^2$, $R^3$, $R^4$ und $R^5$die oben angegebene Bedeutung haben,
mit einem Nitrierungsmittel gegebenenfalls in Gegenwart eines Verdünnungsmittels und gegebenenfalls in Gegenwart eines Reaktionshilfsmittels umsetzt.

5. Herbizide Mittel, gekennzeichnet durch einen Gehalt an mindesetns einem 1-Arylpyrazol der Formel (I) gemäß den Ansprüchen 1 bis 4.

6. Verfahren zur Bekämpfung von Unkräutern, dadurch gekennzeichnet, daß man 1-Arylpyrazole der Formel (I) gemäß den Ansprüchen 1 bis 4 auf die Unkräuter und/oder ihren Lebensraum einwirken läßt.

7. Verwendung von 1-Arylpyrazolen der Formel (I) gemäß den Ansprüchen 1 bis 4 zur Bekämpfung von Unkräutern.

8. Verfahren zur Herstellung von herbiziden Mitteln, dadurch gekennzeichnet, daß man 1-Arylpyrazole der Formel (I) gemäß den Ansprüchen 1 bis 4 mit Streckmitteln und/oder oberflächenaktiven Substanzen vermischt.

9. N-Aryl-N′-cyanethylhydrazine der Formel (II),

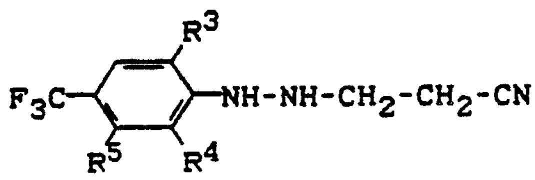 (II)

in welcher
$R^3$ für Wasserstoff oder Halogen steht,
$R^4$ für Halogen steht und
$R^5$ für Wasserstoff oder Halogen steht,

10. Verfahren zur Herstellung von N-Aryl-N′-cyanethlyhydrazinen der Formel (II),

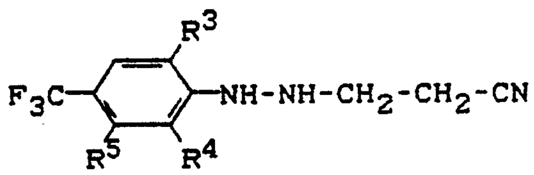 (II)

in welcher
$R^3$ für Wasserstoff oder Halogen steht,
$R^4$ für Halogen steht und
$R^5$ für Wasserstoff oder Halogen steht,
dadurch gekennzeichnet, daß man 1-Arylhydrazine der Formel (IV),

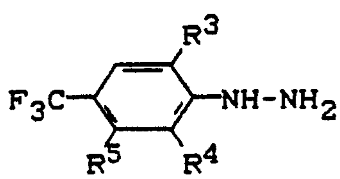 (IV)

in welcher
$R^3$, $R^4$ und $R^5$ die oben angegebenen Bedeutung haben,
mit Acrylnitril der Formel (V),

$CH_2=CH-CN$ (V)

gegebenenfalls in Gegenwart eines Verdünnungsmittels bei Temperaturen zwischen 50° C und 150° C umsetzt.